# EUROPÄISCHE PATENTSCHRIFT

(11) **EP 1 348 761 B1**
(45) Veröffentlichungstag und Bekanntmachung des Hinweises auf die Patenterteilung: **14.11.2007**
(21) Anmeldenummer: 03005228.6
(22) Anmeldetag: 10.03.2003
(51) Int. Cl.: C12N 9/64, C12Q 1/56

(54) **Marburg I Mutante der Faktor VII aktivierenden Protease (FSAP) als Risikofaktor für arterielle Thrombose**
Marburg I mutant of factor VII activating protease (FSAP) as a risk factor for arterial thrombosis
Le mutant Marburg I de la protéase activant le facteur VII (FSAP) comme facteur de risque pour la thrombose arterielle

(30) Priorität: 19.03.2002 DE 10212246; 16.08.2002 DE 10238429
(43) Veröffentlichungstag der Anmeldung: 01.10.2003
(73) Patentinhaber: CSL Behring GmbH, 35041 Marburg (DE)
(72) Erfinder: Kiechl, Stefan, 6170 Zirl (AT); Willeit, Johann, 6020 Innsbruck (AT); Wiedermann, Christian Josef, 6020 Innsbruck (AT); Römisch, Juergen, Dr., 2331 Vösendorf (AT); Weimer, Thomas, 35075 Gladenbach (DE); Feussner, Annette, 35043 Marburg (DE); Stoehr, Hans-Arnold, 35083 Wetter (DE); Doersam, Volker, 35041 Marburg (DE)

(56) Entgegenhaltungen:
- EP-A- 1 059 359
- ROEMISCH J ET AL: "Quantitation of the factor VII- and single-chain plasminogen activator-activating protease in plasmas of healthy subjects." BLOOD COAGULATION & FIBRINOLYSIS, Bd. 12, Nr. 5, Juli 2001 (2001-07), Seiten 375-383, XP009013812 ISSN: 0957-5235
- CHOI-MIURA N H ET AL: "PURIFICATION AND CHARACTERIZATION OF A NOVEL HYALURONAN-BINDING PROTEIN (PHBP) FROM HUMAN PLASMA: IT HAS THREE EGF, A KRINGLE AND ASERINE PROTEASE DOMAIN, SIMILAR TO HEPATOCYTE GROWTH FACTOR ACTIVATOR" JOURNAL OF BIOCHEMISTRY, JAPANESE BIOCHEMICAL SOCIETY, TOKYO, JP, Bd. 119, Nr. 6, 1996, Seiten 1157-1165, XP000960750 ISSN: 0021-924X
- CHOI-MIURA NAM-HO ET AL: "Proteolytic activation and inactivation of the serine protease activity of plasma hyaluronan binding protein." BIOLOGICAL & PHARMACEUTICAL BULLETIN, Bd. 24, Nr. 5, Mai 2001 (2001-05), Seiten 448-452, XP002248254 ISSN: 0918-6158
- ROEMISCH J ET AL: "A PROTEASE ISOLATED FROM HUMAN PLASMA ACTIVATING FACTOR VII INDEPENDENT OF TISSUE FACTOR" BLOOD COAGULATION & FIBRINOLYSIS, RAPID COMMUNICATIONS, OXFORD,OXFORD, GB, Bd. 10, Nr. 8, Dezember 1999 (1999-12), Seiten 471-479, XP001036685 ISSN: 0957-5235
- ROEMISCH J ET AL: "THE FVII ACTIVATING PROTEASE CLEAVES SINGLE-CHAIN PLASMINOGEN ACTIVATORS" HAEMOSTASIS, BASEL, CH, Bd. 29, Nr. 5, 1999, Seiten 292-299, XP001053403
- WILLEIT JOHANN ET AL: "Marburg I polymorphism of factor VII--activating protease: a prominent risk predictor of carotid stenosis." CIRCULATION. UNITED STATES 11 FEB 2003, Bd. 107, Nr. 5, 11. Februar 2003 (2003-02-11), Seiten 667-670, XP009013816 ISSN: 1524-4539
- ROEMISCH JUERGEN: "Factor VII activating protease (FSAP): A novel protease in hemostasis." BIOLOGICAL CHEMISTRY, Bd. 383, Nr. 7-8, Seiten 1119-1124, XP009013814 ISSN: 1431-6730
- ROEMISCH J ET AL: "The frequent Marburg I polymorphism impairs the pro-urokinase activating potency of the factor VII activating protease (FSAP)." BLOOD COAGULATION & FIBRINOLYSIS, Bd. 13, Nr. 5, Juli 2002 (2002-07), Seiten 433-441, XP009013813 July, 2002 ISSN: 0957-5235

## Beschreibung

Die Erfindung betrifft Verfahren zum Nachweis von Mutanten der den Faktor VII aktivierenden Protease (FSAP) sowie von emiedrigten Blutplasmaspiegeln der FSAP als Indikatoren eines erhöhten Risikos zur Entwicklung und Progression von Atherosklerose und der sich daraus ergebenden pathophysiologischen Folgeerscheinungen.

Atherosklerose ist eine krankhafte Veränderung der Arterien, die u.a. mit ihrer Verhärtung, Verdickung und Elastizitätsverlust verbunden ist und als die Hauptursache für Herzinfarkt und Hirnschlag sowie andere Erkrankungen anzusehen ist. Zahlreiche exogene und endogene Faktoren werden für die Auslösung und Förderung der Atherosklerose verantwortlich gemacht, zum Beispiel Hypertonie, Hyperlipidämie, Diabetes, Toxine, Nikotin, exzessiver Alkoholgenuss und Entzündungen. Diese Einflüsse werden als Risikofaktoren bezeichnet. Mit fortschreitender Anzahl von Studien und verbesserten analytischen Mehoden sind jedoch in den letzten Jahren noch weitere Risikofaktoren für Atherosklerose ermittelt worden.

Risikofaktoren werden in epidemiologischen Studien untersucht, wie in der in Fachkreisen bekannt gewordenen Bruneck-Studie. Im Jahre 1990 wurden 1.000 Einwohner von Bruneck/Italien in diese Studie einbezogen. Ultraschall-Untersuchungen der Arteria carotis sowie Analysen einer Anzahl von Blutparametern und die Befragung der Probanden ermöglichte die Etablierung einer breiten Datenbasis für die weitere Verfolgung der Atherosklerose-Entstehung und -Progression. In Abständen von 5 Jahren wurden diese Untersuchungen an denselben Probanden fortgesetzt und analysiert. Hieraus wurde ein Modell der Atheroskleroseentstehung und ihrer Progression abgeleitet. Als ein erstes Ergebnis dieser Studie wurde ein Zusammenhang der Entstehung der Atherosklerose mit den bekannten, traditionellen Risikofaktoren wie der vorstehend genannte Hyperlipidämie und anderen Faktoren festgestellt. Erreicht der atherosklerotische Plaque jedoch ein Ausmaß, dass das Blutgefäß bereits bis zu 40% verschlossen wird, treten andere Risikofaktoren in den Vordergrund, die den weiteren Verlauf der Atherosklerose und des Gefäßverschlusses signifikant beeinflussen können. Zu diesen Faktoren zählen vor allem plasmatische Proteine, die in die Hämostase eingreifen. Hierzu trägt ein vermindertes gerinnungs-inhibitorisches Potential bei, z. B. verminderte Antithrombin- oder Protein C-Spiegel oder auch die sog. APC-Resistenz (aktiviertes Protein C). Deshalb kann die Verminderung des fibrinolytischen Potentials einen entscheidenden Einfluss auf die Progression des Gefäßverschlusses haben, wie es beispielsweise bei erhöhten Blutspiegeln des Lipoproteins (a) beobachtet wird.

Bisher sind vor allem Risikofaktoren von venösen Verschlusskrankheiten identifiziertworden, wie die APC-Resistenz (FV-Leiden), die eine sog. Odds-Ratio bei Heterozygoten für diese Mutation, also ein Maß für die Erhöhung des Risikos, von "8" hat, verglichen mit Probanden ohne eine APC-Resistenz. Im arteriellen Bereich hat dagegen dieser Risikofaktor nur eine Odds-Ratio von ungefähr "2".

Die aus der Bruneck-Studie zur Vertagung stehenden Plasmaproben und DNAs der Probanden wurden deshalb noch einmal auf das Vorhandensein anderer Atherosklerose-Risikofaktoren untersucht und dabei das besondere Augenmerk auf kürzlich aufgefundene Mutanten der den Blutgerinnungsfaktor VII aktivierenden Protease (=FSAP) gerichtet, die im folgenden als FSAP Marburg I Mutation bezeichnet werden.

Aus der deutschen Patentanmeldung 199 03 693.4 ist eine aus dem Blutplasma isolierbare Protease bekannt, die den Blutgerinnungsfaktor VII aktivieren kann. Diese Protease wird auch als Faktor Sieben Aktivierende Protease (FSAP) bezeichnet (oder als PHBP oder PHBSP, entsprechend Plasma Hyaluronsäure-bindende (Serin-) Protease). Deshalb hat FSAP gerinnungsfördernde Eigenschaften. FSAP hat im Besonderen die Eigenschaft, Einketten-Plasminogen-Aktivatoren, wie die Prourokinase oder Einketten-Gewebe-Plasminogen-Aktivator (sct-PA), zu aktivieren. Allein oder in Kombination mit Plasminogen-Aktivatoren kann FSAP aber entsprechend auch zur Unterstützung der Fibrinolyse Anwendung finden, beispielsweise bei thrombotischen Komplikationen.

Die inzwischen zur Verfügung stehenden und in den deutschen Patentanmeldungen 199 03 693.4 und 199 26 531.3 beschriebenen Testsysteme ermöglichen nicht nur die Detektion von FSAP, sondern auch die Quantifizierung des FSAP-Antigengehaltes sowie die Bestimmung ihrer Aktivität im Plasma. Die Antigenbestimmung wird dabei vorzugsweise mittels eines ELISA-Tests durchgeführt. Die Aktivität dagegen kann prinzipiell durch Aktivierung von Prourokinase zur Urokinase und der Umsetzung eines chromogenen Substrats mit anschließender Differenzmessung der Extinktion gemessen werden.

Aus der deutschen Patentanmeldung 100 52 319.6 ist bekannt, dass durch Anwendung dieser Testsysteme bei Untersuchungen von gesunden Blutspendern 5 bis 10% Probanden identifiziert werden konnten, die bei einem durchschnittlichen FSAP Antigengehalt ein deutlich vermindertes Potential zur Aktivierung von Prourokinase aufwiesen. Da diese Eigenschaft auch für die isolierten, individuellen Proteasen zutraf, wurden die entsprechenden DNAs zur weiteren Untersuchung aus Blutzellen analysiert. Überraschenderweise konnte dabei besonders eine Mutation (single nucleotide polymorphism; SNP; G/A in Position 1601) identifiziert werden. Diese Modifikation führt im Protein zu einem Aminosäureaustausch Gly/Glu in der Position 511 des reifen Proteins oder in der Aminosäureposition 534 des FSAP-Proenzyms einschließlich des Signalpeptides. Dieser Aminosäureaustausch hat zur Folge, dass FSAP die Fähigkeit zur Aktivierung von Prourokinase zu Urokinase verliert oder zumindest eine erhebliche Aktivitätseinbuße erleidet. Die vorstehend genannte Mutation, genannt FSAP Marburg I, wurde bisher in allen Proben gefunden, die bei durchschnittlichem Antigengehalt eine verminderte Aktivität bei der Bildung von Urokinase aus Prourokinase aufweisen.

Mit Hilfe eines etablierten PCR-Tests für diese Mutation wurden die DNAs der in die Bruneck-Studie eingeschlossenen Probanden untersucht. Dabei wurden in 4,5% aller analysierten Proben die FSAP Marburg 1-Mutation gefunden. Diese Befunde wurden dann mit den im Rahmen der Studie erhobenen individuellen Daten zur Bewertung von Atherosklerose-Entstehung und -Progression bewertet. Dabei wurde gefunden, dass die FSAP Marburg I-Mutation einen Risikofaktor für die Evolution und Progression der arteriellen Thrombose und Atherosklerose darstellt.

Gegenstand der Erfindung ist deshalb ein Atherosklerose-Risikofaktor, der aus einer Mutante der den Blutgerinnungsfaktor VII aktivierenden Protease (=FSAP) besteht. Ein besonders wichtiger Risikofaktor ist dabei offensichtlich die Mutante, bei der das FSAP Proenzym einschließlich des Signalpeptides an der Aminosäureposition 534 einen Gly/Glu-Austausch aufweist.

Die entsprechende DNA-Sequenz des FSAP-Proenzyms einschließlich des Signalpeptids zeigt dabei an der Nukleotidposition 1601 einen G/A-Basenaustausch.

Überraschenderweise korreliert diese Mutation mit einem erhöhten Risiko, besonders arterielle Thrombose zu entwickeln. Eine Odds-Ratio von "6,6" wurde dabei errechnet, also ein Risiko, das auf der arteriellen Seite vergleichbar ist mit dem Risiko der APC-Resistenz im venösen Bereich. Besonders überraschend ist dabei, dass diese Mutation einen unabhängigen Risikofaktor darstellt, also nach Abzug aller bisher bekannten Risikofaktoren einen eigenen, deutlichen Beitrag zur Entstehung und zur Progression der Atherosklerose leistet. Diese Erkenntnis und die Bestimmung besonders der FSAP Marburg I-Mutation kann deshalb die Chancen verbessern, Herzkrankheiten und atherosklerotisch bedingte Gefäßkrankheiten zu erkennen und zu behandeln. So tritt als Folge von Atherosklerose beispielsweise Angina pectoris auf, häufig gefolgt von Herzinfarkten. Abhängig davon, welche Gefäße betroffen sind, wird das durch dieses versorgte Organ in Mitleidenschaft gezogen. Im Fall der Arteria carotis hat dies eine Unterversorgung des Gehirns mit Nährstoffen und Sauerstoff zur Folge und kann Im schlimmsten Fall zu einem Schlaganfall führen. Aber auch andere durch Atherosklerose betroffene Organe, die dem Risiko einer Gefäßverschlusskrankheit und den daraus sich ergebenden Folgen unterliegen, sind durch die FSAP Marburg I-Mutante betroffen. Daraus können sich Probleme bei Nieren-, Leber-, Lungen- und anderen Krankheiten ergeben.

Methoden zur Bestimmung der FSAP Marburg I-Mutante sind bereits In den vorstehend genannten deutschen Patentanmeldungen, vor allem in der deutschen Patentanmeldung 100 52 319.6 beschrieben. Diese Methoden schließen die Aktivitätsmessung der Protease ein, bevorzugt in Kombination mit einem FSAP Antigentest,sowie die Bestimmung der Nukleotidsequenz in dem mutierten Bereich durch geeignete Testsysteme.

Der erfindungsgemäße Atherosklerose-Risikofaktor kann somit als eine Mutante des FSAP beschrieben werden, die die Fähigkeit zur Aktivierung von Einketten-Plasminogen-Aktivatoren verloren hat oder bei der diese Fähigkeit zumindest beeinträchtigt ist. Insbesondere ist der Risikofaktor durch eine Mutante von FSAP gekennzeichnet, die die Fähigkeit zur Aktivierung von Prourokinase verloren hat oder bei der diese Fähigkeit vermindert ist. Eine genetisch bedingte Veranlagung zur Entstehung arterieller Atherosklerosen kann somit durch den Nachweis der vorstehend genannten Mutanten des FSAP erkannt werden. Insbesondere auch die Veranlagung zur Entstehung besonders arterieller Thrombosen sowie die Veranlagung zur Entstehung von atherosklerotischen oder thrombotischen Erkrankungen und deren Folgeerscheinungen wie arterielle und venöse Verschlusskrankheiten werden durch den Nachweis der genannten FSAP-Mutanten angezeigt. Die Veraniagung zur Entstehung von atherosklerotisch oder thrombotisch bedingten Einschränkungen von Organfunktionen ist eine häufige Ursache für Angina pectoris, Herzinfarkt oder Schlaganfälle. Typisch ist in allen Fällen, dass das Potential zur Aktivierung von Einketten-Plasminogen-Aktivatoren, insbesondere Potential zur Aktivierung von Prourokinase, reduziert ist. Die Reduktion dieses Aktivierungspotentials kann im Blut, vor allem aber im Plasma nachgewiesen werden.

Angesichts der großen Bedeutung der FSAP-Mutanten als Atherothrombose-Risikofaktoren kommen diagnostischen Verfahren zu ihrem Nachweis große Bedeutung zu. Sie beruhen darauf, dass man in den Körperflüssigkeiten des Spenders, insbesondere im Blutplasma, eine verminderte FSAP-Antigenkon- zentration und/oder eine verminderte Aktivität von FSAP bestimmt. Dabei wird in den genannten Körperflüssigkeiten das Potential zur Aktivierung von Einketten-Ptasminogen-Aktivatoren, insbesondere das Potential zur Aktivierung von Prourokinase, bestimmt.

Ein besonderszuverlässiges Verfahren besteht darin, dass hetero- oder homozygote Mutanten des FSAP-Proenzyms mit einem G/A-Basenaustausch an der Nukleotidposition 1601 durch Analyse der genomischen DNA des Spenders oder in einer davon abgeleiteten mRNA nachgewiesen werden.

Ebenso ist es aber auch möglich, die genannten FSAP-Mutanten auf der Proteinebene nachzuweisen. Hierfür eignet sich vor allem die Anwendung von spezifischen monoklonalen oder polyklonalen Antikörpern. Schließlich stehen aber auch histologische Untersuchungsmethoden an Geweben oder in aus Geweben extrahierten Lösungen zur Verfügung.

Ein zuverlässiges diagnostisches Verfahren besteht darin, dass man eine Probe, die die FSAP Mutante(n) enthalten könnte, mit einem auf einem festen Träger fixierten ersten Antikörper inkubiert und danach wäscht, dann einen zweiten, markierten Antikörper oder einen gegen den Wildtyp gerichteten markierten Antikörper zugibt, abermals auswäscht und das vom zweiten Antikörper hervorgerufene Signal misst.

Eine andere Methode besteht darin, das man eine Probe, die die FSAP Mutante(n) enthalten könnte, mit einem auf einem festen Träger fixierten ersten Antikörper gegen den Wildtyp Inkubiert und danach wäscht, dann einen zweiten, markierten Antikörper zugibt, abermals auswäscht und das vom zweiten Antikörper hervorgerufene Signal misst.

Weiterhin steht ein Verfahren zur Verfügung, bei dem man auf einem Träger die auf das Vorliegen der FSAP Mutante(n) zu untersuchende Probe fixiert und sie mit einem markierten Antikörper allein oder in Mischung mit einem unmarkierten Antikörper und anschließendem Nachweis des markierten Antikörpers detektiert.

Schließlich kann man auch einen auf einem Träger fixierten Antikörper mit einer auf das Vorliegen der FSAP Mutante(n) zu untersuchenden Probe in Gegenwart einer markierten Mutante versetzen und das durch die Markierung hervorgerufene Signal messen.

Bewährt hat sich auch ein diagnostisches Verfahren, bei dem man die Aktivität der FSAP misst, indem man die die Protease enthaltende Probe an einem festen Träger inkubiert, an den zuvor gegen die Protease gerichteter Antikörper gekoppelt wurde und dann nach dem Auswaschen des freien Trägers die daran fixierte Protease mit Reagentien inkubiert, die deren Aktivitätsbestimmung erlauben.

Weiterhin stehen diagnostische Verfahren zur Verfügung, bei denen man Antikörper zur Detektion der Mutanten auf Westernblots zur Immunhistologie oder des "fluoreszenz-activated Cell Sorting (FACS)" verwendet.

Vorzugsweise wird das erfindungsgemäße diagnostische Verfahren mit der ELISA-Technikdurchgeft7hrt. Dabei wird FSAP und/oder die FSAP-Mutante an eine Matrix gebunden, zum Beispiel an eine Mikrotiterplatte. Zur optimalen Präsentation des FSAP und/oder der FSAP-Mutante kann zuvor eine Beschichtung der Platte mit monoklonalen oder polyklonalen Antikörpern oder deren F(ab')₂ oder Fab' vorgenommen werden, um danach mit FSAP und/oder der FSAP-Mutante beladen zu werden. Da FSAP und die Mutante sehr gut an Dextransulfat, Heparin und ähnliche Substanzen binden, ist auch die vorherige Beschichtung mit diesen Agentien zur FSAP-Bindung möglich. Nach Waschen des Trägers oder der Mikrotiterplatte wird diese ggf. mit den hierfür bekannten Agentien wie Detergenz oder Albumin, blockiert, gewaschen und anschließend mit der zu testenden Lösung inkubiert. Bei den FSAP-Antikörpern enthaltenden Lösungen kann es sich um Blutserum, Plasma und andere Körperflüssigkeiten wie auch Synovialflüssigkeiten, Liquor, Speichel, Tränen, Seminalplasma oder auch Zelllysate handeln.

Nach Inkubation und Waschen des Trägers findet dann ein geeignetes Nachweisreagenz Verwendung. Die zur Detektion der verschiedenen Antikörperklassen wie IgG, IgM, IgA, IgE und den dazugehörigen Subklassen erforderlichen Testsubstanzen sind als markierte Reagentien im Handel erhältlich. Der Nachweis und die Quantifizierung des Antikörpertiters kann dann durch eine photometrische Bestimmung erfolgen, bei der die Extinktion gemessen wird, die durch die Spaltung eines chromogenen Substrats durch ein an den Anti-Human-Antikörper gekoppeltes Enzym bewirkt wird. Es ist aber auch möglich, die Fluoreszenz zu messen, die die mit dem zum Nachweis verwendeten Antikörper verbundene fluoreszierende Gruppe ausstrahlt Schließlich ist es auch möglich, den Nachweis mit einer radiometrischen Messung durchzuführen, wenn die zur Detektion verwendete Substanz mit einer radioaktiven Gruppe markiert ist. Diagnostische Verfahren, bei denen die gebundenen Human-Antikörper mit einem markierten Anti-Humanlmmunglobulin oder deren Fragmenten oder markierten Protein A oder Protein G inkubiert werden und bei denen das von der gebundenen, markierten Substanz ausgesendete Signal bestimmt wird, haben sich schon vielfach gut geeignet erwiesen.

Ebenso ist es möglich, die Antikörper durch eine photometrische Messung der Extinktion nachzuweisen, die durch die mit einem Enzym gekoppelten Anti-Human-Antikörper oder deren Fragmente oder Protein A oder Protein G durch Spaltung eines geeigneten chromogenen oder fluorogenen Substrats verursacht wird. Geeignet sind auch diagnostische Verfahren, bei denen man die Antikörper durch die Messung der Fluoreszenz nachweist, die von der mit fluoreszierenden Gruppen markierte gebundene Substanz verursacht wird.

Insbesondere sind daher Gegenstand der vorliegenden Erfindung Verfahren zur Erkennung eines erhöhten Risikos der Entstehung und Progression arterieller Thrombosen und atherosklerotischer Erkrankungen und deren Folgeerkrankungen wie beispielsweise Angina Pectoris, Herzinfarkt und Schlaganfall, **dadurch gekennzeichnet, dass** in Proben von Körperflüssigkeiten eines Spenders, insbesondere im Blutplasma entweder
a) das hetero- oder homozygote Vorkommen einer Mutante des FSAP Proenzyms mit einem G/A Basenaustausch an der Nukleotidposition 1601 durch Analyse der genomischen DNA des Spenders oder in einer davon abgeleiteten mRNA nachgewiesen wird
   **und/oder**
b) das Vorkommen einer FSAP Mutante auf Proteinebene nachgewiesen wird, die die Fähigkeit zur Aktivierung von Einketten-Plasminogen-Aktivatoren verloren hat oder bei der diese Fähigkeit zumindest beeinträchtigt ist,
   **und/oder**
c) das Vorliegen spezifischer Antikörper gegen eine FSAP Mutante, die die Fähigkeit zur Aktivierung von Einketten-Plasminogen-Aktivatoren verloren hat oder bei der diese Fähigkeit zumindest beeinträchtigt ist, nachgewiesen wird
   **und/oder**
d) ein vermindertes Potential der FSAP zur Aktivierung von Einketten-Plasminogen-Aktivatoren nachgewiesen wird
   **und/oder**
e) eine verminderte Antigenkonzentration der FSAP nachgewiesen wird.

### Bruneck Studie

Studienprobanden: Bei der Bruneck-Studie handelt es sich um eine prospektive Populationsuntersuchung, die darauf zielt, Epidemiologie und Ätiologie von Carotis-Atherosklerose zu erhellen (1-6). Die Studienpopulation wurde 1990 als nach Geschlecht und Alter geschichtete Stichprobe aller Einwohner von Bruneck im Alter von 40 bis 79 Jahren (aus den Altersdekaden 5 bis 8 jeweils 125 Frauen und 125 Männer, n=1000) rekrutiert. Insgesamt nahmen 93,6% teil, wobei 919 die Datenerhebung absolvierten. Während der Nachuntersuchungsperiode zwischen Sommer 1990 und 1995 (Quinquennium₁ - Q₁) verstarb eine Untergruppe von 62 Individuen, während ein Proband verzog. Follow-up in der verbliebenen Population war zu 96,5% komplett (n=826) (1-3). Vor der Aufnahme in die Studie gaben alle Teilnehmer, nachdem sie über die Studie informiert worden waren, ihre Einwilligung. Als Teil der Nachuntersuchung 1995 wurden Blutproben für die DNA-Gewinnung entnommen. In 16 Fällen wurden keine zufriedenstellenden PCR-Produkte erhalten, d.h. für die Hauptanalyse verblieben 810 Männer und Frauen. Von diesen Probanden verstarben zwischen Sommer 1995 und 2000 (Quinquennium₂ - Q₂) 94. Insgesamt 675 Probanden wurden 2000 nochmals ultraschalluntersucht (Follow-up-Quote bei den Überlebenden 94,3%) (6).

Klinische Anamnese und Untersuchung: Zum Studienprotokoll gehörte eine klinische Untersuchung mit kardiologischen und neurologischen Schwerpunkten und standardisierten Fragebögen zur gegenwärtigen oder vergangenen Gefährdung durch potentielle Gefäßrisikofaktoren (3-5). Bei Rauchern und ehemaligen Rauchern wurde die pro Tag durchschnittlich gerauchte Anzahl an Zigaretten und die Packungsjahre festgehalten. Der Alkoholkonsum wurde als Gramm pro Tag quantifiziert und in vier Kategorien eingeteilt (3). Systolischer und diastolischer Blutdruck waren die Mittelwerte von drei Messungen, die jeweils nach ≥ 10 Minuten Ruhe gemessen wurden. Bluthochdruck wurde als ein Blutdruck von ≥ 160/95 bzw. Einnahme von blutdrucksenkenden Mitteln (WHO-Definition) definiert. Bei allen Probanden mit Ausnahme der vorbekannten Diabetiker wurde ein standardisierter oraler Glucosetoleranztest durchgeführt. Diabetes mellitus wurde bei denjenigen Probanden als vorliegend eingetragen, die Nüchterrtblutzuckerspiegel von ≥ 140 mg/dl (7,8 mmol/l) und/oder einen 2-Stunden-Wert (oraler Glucosetoleranztest) ≥ 200 mg/dl (11,0 mmol/l) aufwiesen.

Labormethoden: Nachdem die Probanden wenigstens 12 Stunden lang keine Nahrung zu sich genommen und nicht geraucht hatten, wurden aus der Ellenbeugenvene Blut entnommen (3-6). Gesamtcl10lesterin und Cholesterin mit Lipoprotein hoher Dichte wurden enzymatisch bestimmt (CHOD-PAP-Methode, Merck, Darmstadt, Deutschland), und Lipoprotein(a)-Konzentrationen wurden mit einem ELISA gemessen (Immuno, Wien, Österreich). Das Cholesterin mit Lipoprotein niedriger Dichte wurde über die Friedewald-Formel berechnet. Fibrinogen wurde gemäß der Methode von Clauss und Antithrombin III mit einem chromogenen Assay gemessen. Die Leiden-Mutation des Faktors V wurde durch alleispezifische PCR-Amplifikation nachgewiesen (3).

FSAP-Antigenkonzentrationen und scuPA-aktivierende Wirkung wurden wie vor kurzem beschrieben bestimmt (7, 8). Kurz gesagt wurde ein ELISA mit monoklonalen Antikörpern (mAb) gegen FSAP zur Antigenquantifizierung verwendet. Der Assay auf Aktivität beinhaltete eine Immunadsorption an mit Antikörpern beschichteten Mikrotiterplatten, einen Waschschritt und die anschließende Aktivierung von Prourokinase durch FSAP, die durch photometrische Beobachtung der Amidolyse eines chromogenen Substrats für Urokinase quantifiziert wurde. Als willkürlicher Standard für beide Assays diente ein Plasmapool von mehr als 200 gesunden Blutspendern. Eine Plasmaäqulvalenteinheit (PÄE) wurde als die in einem Milliliter des Plasmapools enthaltene FSAP-Antigenaktivität definiert, die im Durchschnitt 12 µg/ml entspricht (8).

DNA-Extraktion und FSAP-Genotyping: Qualitativ hochwertige DNA wurde mit einem GenomicPrep Blood DNA Isolation Kit (Amersham Pharmacia Biotech) aus gefrorenem Vollblut erhalten. Zehn µl extrahierte DNA wurden in 100 µl 1x PCR-Standardreaktionspuffer mit 50 pmol des entsprechenden exonspezifischen Vorwärts- und Rockwärtsprimers,1,5 mM MgCl₂, 0,2 mM dNTP und 2,5 Einheiten Taq-DNA-Polymerase (Perkin Eimer, Langen, Deutschland) amplifiziert; einer ersten 2minütigen Denaturierung bei 94°C folgten 35 Thermozyklen mit jeweils 30-Sekunden bei 94°C, 30 Sekunden bei 50°C und 40 Sekunden bei 72°C, woran sich ein abschließender Elongationsschritt von 5 Minuten bei 72°C anschloß. Die verwendeten Primer-Paare wurden vor kurzem ausführlich beschrieben.

Scanning-Protokoll und Definition der Ultraschall-Endpunkte: Bei der Ultraschalluntersuchung wurden A. carotis interna (bulböse und distale Abschnitte) und A. carotis communis (proximale und distale Abschnitte) beider Seiten mit einem 10-MHz-Schallkopf und einem 5-MHz-Doppler (1,2) gescannt. Atherosklerotische Läsionen wurden durch zwei Ultraschallkriterien definiert: 1) Wandoberfläche (Herausragen in das Lumen) und 2) Struktur der Wand (Echogenität). In 16 Gefäßabschnitten wurde jeweils der maximale axiale Plaque-Durchmesser bestimmt (Intraobservationskoeffizient der Variation je nach Gefäßabschnitt 10% bzw. 15%). Die Dicke der Intima media wurde an den entfernt liegenden Wänden der A. carotis communis gemessen (Intraobservationskoeffizient der Variation 7,9% (n=100)) (2). Die Scans wurden in den Jahren 1990, 1995 und 2000 vom selben erfahrenen Ultraschallspezialisten durchgeführt, wobei dem Ultraschallspezialisten die klinischen Befunde und Laborwerte der Probanden nicht bekannt waren.

Das Entstehen von Atherosklerose war durch das Auftreten von neuen Plaques in zuvor normalen Abschnitten charakterisiert. Bei der Definition von entstehender Atherosklerose wurden Schwellenwerte von 0,7 mm (A. carotis communis) bzw. 1,0 mm (A. carotis interna) als Minimalanforderungen bezüglich der Plaquedurchmesser eingeführt, da kleinere Läsionen schwer von fokalen/diffusen Wandverdickungen zu unterscheiden waren (1). Ein Fortschreiten von nicht-stenotischen Läsionen wurde als relative Vergrößerung des Plaque-Durchmessers von mehr als zweimal dem Meßfehler der Methode definiert (1). Bei der gegenwärtigen Analyse wurden die beiden Vorgänge zu einer einzigen Ergebniskategorie zusammengefaßt, die zur einfacheren Darstellung und aufgrund der Tatsache, daß bei diesen Vorgängen die meisten der beschriebenen Risikofaktoren gemeinsam vorlagen, als "frühe Atherogenese" bezeichnet wurde. Eine "fortgeschrittene Atherogenese" wurde immer dann angenommen, wenn das Fortschrittskriterium erfüllt wurde und das Lumen um > 40% verengt war. Wie andernorts beschrieben (1-5), schien der Schnitt bei 40% einem biologischen Schwellenwert in unserer Population zu entsprechen, bei dem deutliche Änderungen in der Wachstumskinetik der Plaques (kontinuierliches, langsames und diffuses Wachstum kontra gelegentlichen und fokalen Expansionen prominenter Läsionen), bei den Risikoprofilen (gewöhnliche Risikofaktoren kontra Prokoagulationsrisikofaktoren) und beim Erneuerungsprozeß in den Gefäßen (kompensierend bzw. überkompensierend kontra unzureichend oder sogar abwesend) auftraten, die indikativ für eine Umstellung der zugrundeliegenden pathogenen Mechanismen von gewöhnlicher Atherogenese zu Atherothrombose waren.

Die Reproduzierbarkeit der Ultraschallkategorien war "nahezu perfekt" (kappa-Koeffizienten von >0,8, erhalten aus zwei unabhängigen Messungen, die vom selben Ultraschallspezialisten in einer Stichprobe zur Reproduzierbarkeit von n = 100 (1-3) durchgeführt wurde).

Statistische Analyse: Mögliche Assoziationen zwischen FSAP-Mutationen und den verschiedenen Atherogenesestadien wurden mittels logistischer Regressionsanalyse untersucht. Ein Basismodell wurde lediglich in bezug auf Alter und Geschlecht angeglichen. Multivariate Gleichungen wurden durch einen schrittweise fortschreitenden Selektionsvorgang angepaßt, wie bereits beschrieben (p-Werte für Aufnahme und Ausschluß 0,10 bzw. 0,15) (3, 10). Alter und Geschlecht wurden zusätzlich in diese Modelle eingeführt, um die Alters- und Geschlechtsstruktur der Stichprobe aus der Bevölkerung mit zu berücksichtigen. Die Hauptanalyse konzentrierte sich auf den Zeitraum zwischen 1990 und 1995 (Q₁). Die Analyse bei fortgeschrittener Atherogenese beschränkte sich auf Probanden, die zu Beginn der Studie bereits an Atherosklerose litten (n=326).

Die regressionsstandardisierten Atherogeneserisiken wurden für eine Reihe von Risikofaktoren berechnet. Zur Anwendung kam die Randmethode des Regressionsangleichungsverfahrens, da hierbei nicht von der Annahme seltener Krankheiten ausgegangen wird (11).

### ERGEBNISSE

Aus der Bruneck-Studienkohorte (n=810) waren 36 Probanden heterozygot für die Marburg I Mutante von FSAP (17 Männer und 20 Frauen) und ein Proband war homozygot, was einer Gesamtträgerquote [95% VI] in der Allgemeinbevölkerung von 4,4% [3,0% bis 5,8%] entspricht. Eine Kosegregation der Marburg II Mutante (E370Q) von FSAP wurde in 16 der 37 Individuen (43 Prozent, 8 Männer und B Frauen) beobachtet, während bei den verbliebenen 21 Probanden (57 Prozent, 9 Männer und 12 Frauen) die Marburg I Mutante isoliert auftrat.

Plasmaproben einer Teilpopulation (n=82) wurden auf FSAP-Antigenkonzentrationen und entsprechende prourokinase-aktivierende Wirkungen untersucht. Bei 76 Probanden mit Wildtyp-FSAP betrugen die mittleren (±2xSD) Antigenkonzentrationen, Aktivitätskonzentrationen und Aktivitäts/Antigen-Verhältnisse 0,991 (0,552 bis 1,430) PÄE/ ml, 1,036 (0,614 bis 1,458) PÄE/ml bzw. 1,07 (0,63 bis 1,51). Im Gegensatz dazu zeigten alle sechs Träger der Marburg I Mutante in dieser Untergruppe eine deutlich reduzierte in-vitro-Kapazität zur Aktivierung von Prourokinase (<0,150 bis 0,626) und Aktivitäts/Antigen-Verhältnisse von 0,38 bis 0,58. Bei der Verteilung dieser Parameter in den beiden genetischen Gruppen gab es kaum eine Überschneidung.

Während der fünfjährigen Nachuntersuchungsperiode zwischen 1990 und 1995 (Q₁) kam es bei insgesamt 384 der 810 Probanden in der Studie (47,4%) zur Entstehung neuer atherosklerotischer Läsionen bzw. zu einer Ausweitung nichtstenotischer Läsionen (frühe Atherogenese), und bei 92 von 326 Individuen (28,2%) mit bereits vorhandenen Plaques zeigten sich stenotische Transformationen (fortgeschrittene Atherogenese). Wie erwartet wurde kein Zusammenhang zwischen Marburg l Mutante und früher Atherogenese gefunden (alters/geschlechtsangspaßt multivariate Chancenverhältnisse [95% VI] von 0,6 [0,3 bis 1,4] bzw. 0,7 [0,3 bis 1,7]. Im Einklang damit gab es zwischen den Trägern von Wildtyp-FSAP (0,95 mm) und der Marburg l Mutante von FSAP (0,94 mm; P=0,853 für die Differenz) keine Unterschiede bei der Dicke der Intima media der A. carotis communis. Es stellte sich jedoch heraus, daß die Mutante ein starker Risikofaktor für das fortgeschrittene putative Atherothrombosestadium bei der Atherogenese ist (alters-/geschlechtsangepaßtes Chancenverhältnis [95% VI] 3,5 [1,1 bis 11,4], P=0,036). Der Zusammenhang blieb bei Anpassung des logistischen Regressionsmodells auf andere relevante Risikofaktoren statistisch signifikant (Tab. 1). Zum Risikoprofil für fortgeschrittene stenotische Atherosklerose gehörten ferner Diabetes, eine hohe Fibrinogenkonzentration, eine niedrige Antithrombinkonzentration, eine hohe Thrombozytenzahl, Rauchen, Alkoholkonsum (geringe Mengen protektiv), Lp(a) >0,32 g/l und Leiden-Mutation des Faktors V. Bei den Risikofaktoren gab es keine geschlechtsspezifischen Unterschiede, und Belege für differentielle Wirkungen der Marburg I Mutante in nach Alter, Risikohöhe und Lebensgewohnheiten geordneten Teilpopulationen wurden nicht gefunden. Ein Ausschluß von Probanden, die Aspirin, Mittel gegen Bluthochdruck, Antidiabetika oder Lipidsenker einnahmen, beeinflußte die Ergebnisse ebenfalls nicht. Die regressionsstandardisierten Risiken fortgeschrittener Atherogenese für eine Reihe von Hauptrisikofaktoren (Marburg I Mutante, IGT/Diabetes, hohe Lipoprotein(a)-Konzentration, Rauchen, Faktor V Mutation, hohe Fibrinogenkonzentration und niedrige Antithrombinkonzentration) sind in Tabelle 2 angeführt. Bei Probanden mit keinem der Risikofaktoren bestand ein geringes Risiko für das Entstehen/Fortschreiten von Carotisstenose, während es bei Probanden miteinem Cluster von mehr als zwei Faktoren schon fast zwingend zu fortgeschrittener Atherogenese kam.

Die Marburg II Mutante hatte keine Wirkung auf die in-vitro-Aktivierung von Einketten-Plasminogen-Aktivatoren durch FSAP. Dementsprechend war es nicht unerwartet, daß in unseren Analysen kein Zusammenhang zwischen dieser Mutation und Atherogenese gefunden werden konnte. Beim Vergleich von Probanden mit Wildtyp-FSAP und Trägern der Marburg II Mutante, der Marburg I Mutante und Trägern beider genetischer Abweichungen betrugen die multivariaten Chancenverhältnisse [95% VI] für fortgeschrittene Atherosklerose 1,6 [0,2 bis 19.7]. P=0,669, 6,2 [1,1 bis 36.0], P=0,048. bzw. 7,1 [1,1 bis 45,1]. P=0,037.
Zum Nachweis, daß unsere Befunde auch über längere Zeiträume konsistent sind, wurden die Berechnungen mit den Daten aus der zehnjährigen Nachuntersuchungsperiode zwischen 1990 und 2000 (Q₁₊₂) wiederholt. Bei diesen Gleichungen war die multivariate Beziehung zwischen der Marburg I Mutante von FSAP und fortgeschrittener Atherosklerose (gleiche Anpassungen wie bei der ursprünglichen Analyse) wiederum statistisch signifikant (multivariates Chancenverhältnis [95% VI] 4,1 [1,1 bis 14,8], P=0,045).

**Table 1.**

| Multivariante logistische Regressionsanalyse fortgeschrittener Atherogenese nach Alter, Geschlecht; Marburg I Mutante von FSAP und anderen potentiellen Gefäßrisikofaktoren | | | | | |
|---|---|---|---|---|---|
| **Mittelwerte ± Standardabweichung / (%)** | | | | | |
| **Variable** | **AS - (n=234)** | **AS + (n=92)** | **Chancenverhältnis (95% VI)** | **P-Wert** | **Schritt** |
| Alter, J | 64,9±9,2 | 67,8±8,0 | 1,67 (1,19-2,92) | ,0064 | 0 |
| Weibliches | 109 (46,6%) | 32 (34,8%) | 0,56 (0,25-1,25) | ,1555 | 0 |
| Geschlecht | | | | | |
| | | | | | |
| Glucosetoleranz | | | | <,0001 | 1 |
| IGT | 20 (8,5%) | 16 (17,4%) | 3,31 (1,37-7,99) | ,0081 | |
| DM | 10 (B,1%) | 21 (22,8%) | 6,38 (2,71-14,99) | <,0001 | |
| Zigaretten/Tag | 3,2±7,2 | 6,6±9,6 | 1,77 (1,30-2,40) | ,0003 | 2 |
| Lp(a)>0,32 g/l | 36 (15,4%) | 25 (27,2%) | 4,06 (1,83-8,96) | ,0005 | 3 |
| Alkoholkonsum | | | | ,0043 | 4 |
| <1 g/d | 114 (48,7%) | 42 (45,6%) | 1,00 | | |
| 1-50 g/d | 60 (25,7%) | 15 (16,3%) | 0,26 (0,10-0,66) | ,0046 | |
| 51-99 g/d | 57 (15,8%) | 17 (18,5%) | 1,03 (0,40-2,70) | ,9475 | |
| ≥100 g/d | 23(9,8%) | 18(19,6%) | 1,90(0,63-5,69) | ,2535 | |
| Fibrinogen, g/l | 2,7±0,6 | 2,9±0,6 | 1,53 (1,12-2,09) | ,0083 | 5 |
| **Marburg I FSAP** | **5(2,1%)** | **8(8,7%)** | **6,63 (1,58-27,72)** | ,0099 | 6 |
| **Mutation** | | | | | |
| Faktor VMutation | 5(2,1%) | 7 (7,6%) | 4,70 (1,19-18,55) | ,0291 | 7 |
| Antithrombin III, % | 96,3±13,0 | 92,8±16,4 | 0,74 (0,55-1,00) | ,0500 | 8 |
| Trombozytenzah, x10⁹/l | 217,4±56,5 | 230,3±56,6 | 1,32 (0,98-1,77) | ,0769 | 9 |

Chancenverhältnisse (CV), 95% Vertrauensintervall (95% VI) und p-Werte (P) wurden aus der logistischen Regressionsanalyse von fortgeschrittener Atherosklerose (Entstehen/Fortschreiten von stenotischer Carotisatherosklerose) bezüglich Alter, Geschlecht und Gefäßrisikofaktoren abgeleitet Das Modell wurde durch einen schrittweise fortschreitenden Selektionsvorgang angepaßt (Schritt... Eintrittsschritt). Die CV wurden für eine 1-SD Einheitveränderung gegebener Variablen berechnet. AS-: Gruppe ohne fortgeschfetene Atherogenese, AS+: Gruppe mit fortgeschrittener Atherogenese. Diese Analyse konzentrierte sich auf die 326 Probanden, die 1990 zu Beginn der Studie bereits an Atherosklerose

### LITERATURANGABEN

1. Kiechl S, Willeit J. The natural course of atherosclerosis. Part I: incidence and progression. Arterioscler Thromb Vasc Biol 1999; 19: 1484-90.
2. Kiechl S, Willeit J. The natural course of atherosclerosis. Part II: vascular remodeling. Arterioscler Thromb Vasc Biol 1999:19:1491-8.
3. Willeit J, Kiechl S, Oberhollenzer F, et al. Distinct risk profiles of early and advanced atherosclerosis. Prospective results from the Bruneck Study. Arterioscier Thromb Vasc Biol 2000;20:529-37.
4.Kiechl S, Egger G, Mayr M, et al. Chronic infections and the risk of carotid atherosclerosis. Prospective results from a large population study. Circulation 2001;103:1064-70.
5. Kiechl S, Willeit J, Egger G, Poewe W, Oberhollenze F. Body iron stores and the risk of carotid atherosclerosis. Prospective results from the Bruneck Study. Circulation 1997;96:3300-7.
6. Kiechl S, Lorenz E, Reindl M, Wiedermann CJ, Oberhollenzer F, Nonora E, Willeit J, Sxhartz DA. Toll-like receptor 4 polymorphisms and atherogenesis in humans. *N Engl J Med* 2002;347 (in press).
7. Roemisch J, Feussner A, Stohr HA. Quantitation of the factor VII- and single-chain plasminogen activator-activating protease in plasmas of healthy subjects. Blood Coagul Fibrinolysis. 2001;12:375-83.
8. Kannemeier C, Feussner A, Stohr HA, Weisse J, Preissner KT, Roemisch J. Factor VII and single-chain plasminogen activator-activating protease: activation and autoactivation of the proenzyme, Eur J Biochem. 2001;268:3789-96.
9. Roemisch J, Feussner A, Nerlich C, Stoehr HA, Weimer T. The frequent, Marburg I polymorphism impairs the prourokinase activating potency of the factor VII_activating protease (FSAP). Blood Coag Fibrinol 2002; 13. in press.
10. Hosmer DW, Lemeschow *S. Applied Logistic Regression*, New York: John Wiley & Sons, 1988.
11. Wilcosky TC, Chambless LE. A comparison of direct adjustement and regression adjustement of epidemiologic measures. J Chron Dis 1985;36:649-56.

### LEGEND ZU DER ABBILDUNG

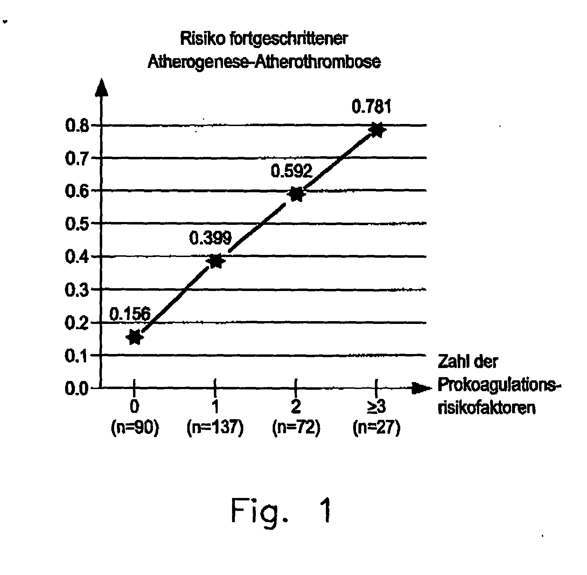

Abbildung 1: Die Abbildung zeigt die regressionangepaßten Risiken fortgeschrittener Atherogenese als Funktion der bei einem Individuum vorhandenen Gefäßrisikofaktoren (Marburg I Mutante der Faktor VII aktivierenden Protease, IGT/Diabetes, Lipoprotein(a)-Konzentration>0,32 g/l, Rauchen, Leiden-Mutation des Faktors V, Fribinogen-konzentration (Q₅, >3,3 g/l) und Antithrombinkonzentration (Q₁, <0,84%)).

## Patentansprüche

1. Verfahren zur Erkennung eines erhöhten Risikos der Entstehung und Progression arterieller Thrombosen und atherosklerotischer Erkrankungen und deren Folgeerkrankungen wie beispielsweise Angina Pectoris, Herzinfarkt und Schlaganfall, **dadurch gekennzeichnet, dass** in Proben von Körperflüssigkeiten eines Spenders, insbesondere im Blutplasma entweder
a) das hetero- oder homozygote Vorkommen einer Mutante des FSAP Proenzyms mit einem G/A Basenaustausch an der Nukleotidposition 1601 durch Analyse der genomischen DNA des Spenders oder in einer davon abgeleiteten mRNA nachgewiesen wird
**und/oder**
b) das Vorkommen einer FSAP Mutante auf Proteinebene nachgewiesen wird, die die Fähigkeit zur Aktivierung von Einketten-Plasminogen-Aktivatoren verloren hat oder bei der diese Fähigkeit zumindest beeinträchtigt ist,
**und/oder**
c) das Vorliegen spezifischer Antikörper gegen eine FSAP Mutante, die die Fähigkeit zur Aktivierung von Einketten-Plasminogen-Aktivatoren verloren hat oder bei der diese Fähigkeit zumindest beeinträchtigt ist, nachgewiesen wird
**und/oder**
d) ein vermindertes Potential der FSAP zur Aktivierung von Einketten-Plasminogen-Aktivatoren nachgewiesen wird
**und/oder**
e) eine verminderte Antigenkonzentration der FSAP nachgewiesen wird.

2. Verfahren nach Anspruch 1, **dadurch gekennzeichnet, dass** ein hetero- oder homozygotes Vorkommen einer Mutante der FSAP nachgewiesen wird, wobei die Mutante der FSAP, einen Aminosäureaustausch von Glycin zu Glutaminsäure an Position 534 der Aminosäuresequenz des FSAP Proenzyms einschließlich des Signalpeptids aufweist.

3. Verfahren nach Anspruch 1 und 2, **dadurch gekennzeichnet, dass** das hetero- oder homozygoten Vorkommen der Mutante durch Analyse der Sequenz der genomischen DNA eines Spenders oder der davon abgeleiteten mRNA in Körperflüssigkeiten nachgewiesen wird, wobei eine Amplifikation mit Hilfe der Polymerase Chain Reaction (PCR) erfolgt.

4. Verfahren nach einem der Ansprüche 1 bis 3, **dadurch gekennzeichnet, dass** ein hetero- oder homozygotes Vorkommen einer Mutante der FSAP, die die Fähigkeit zur Aktivierung von Prourokinase verloren hat oder bei der diese Fähigkeit zumindest beeinträchtigt ist, nachgewiesen wird.

5. Verfahren nach Anspruch 1 bis 4, **dadurch gekennzeichnet, dass** die Mutanten der FSAP auf Proteinebene durch Anwendung von monoklonalen oder polyklonalen Antikörpern nachgewiesen werden.

6. Verfahren nach Anspruch 5, **dadurch gekennzeichnet, dass** die Mutanten der FSAP auf Proteinebene durch Anwendung von monoklonalen oder polyklonalen Antikörpern nachgewiesen werden, welche spezifisch für die Mutante sind.

7. Verfahren nach Anspruch 5 und 6, **dadurch gekennzeichnet, dass** die Mutanten durch histologische Untersuchungen an Geweben oder in aus Geweben extrahierten Lösungen nachgewiesen werden.

8. Verfahren nach Anspruch 1 bis 7, **dadurch gekennzeichnet, dass** der Nachweis einer verminderten FSAP-Antigenkonzentration mittels eines ELISA Verfahrens durchgeführt wird, wobei FSAP aus Körperflüssigkeiten als Protein direkt oder indirekt über an eine feste Phase fixierte FSAP bindende Substanzen wie mono- oder polyklonale Antikörper sowie deren F(ab')₂ oder Fab'-Fragmente, als auch Dextransulfat oder Heparin an eine feste Phase gebunden wird, und die Bestimmung der FSAP entweder durch Bestimmung der Aktivität der gebundenen FSAP erfolgt oder durch Nachweis der Bindung weiterer FSAP spezifischer Bindungspartner.

9. Verfahren nach Anspruch 1 bis 8, **dadurch gekennzeichnet, dass** der Nachweis der FSAP Mutante mittels an eine feste Phase gebundener Antikörper erfolgt, welche mit einer auf das Vorliegen des Risikofaktors zu untersuchenden Probe in Gegenwart einer markierten FSAP Mutante versetzt wird, und das durch die Markierung hervorgerufene Signal gemessen wird.

10. Verfahren nach Anspruch 1 bis 9 **dadurch gekennzeichnet, dass** zum Nachweis eines verminderten Potentials der FSAP zur Aktivierung von Einketten-Plasminogen-Aktivatoren die Aktivität der FSAP gemessen wird, indem die die Protease enthaltende Probe an einem festen Träger inkubiertwird, an den zuvor ein gegen die Protease gerichteter Antikörper gekoppelt wurde, und nach Auswaschen des freien Trägers die daran fixierte Protease mit Reagenzien inkubiert, die deren Aktivitätsbestimmung erlauben.

11. Verfahren nach den Ansprüchen 1 bis 10, **dadurch gekennzeichnet, dass** zum Nachweis des Vorkommens der FSAP-Mutanten Antikörper auf Western Blots zur Immunhistologie oder das fluoreszenz-aktivierte Cell Sorting (FACS) verwendet werden.

12. Verfahren nach den Ansprüchen 1 bis 11, **dadurch gekennzeichnet, dass** man zum Nachweis von Antikörpern gegen eine Mutante der FSAP eine Probe, die die Antikörper gegen die Mutante der FSAP enthalten könnte, auf eine an einen festen Träger fixierte FSAP-Mutante einwirken lässt, danach wäscht und die an die Protease(n) gebundenen Antikörper nachweist.

13. Verfahren nach Anspruch 12, **dadurch gekennzeichnet, dass** die gebundenen Human-Antikörper mit einem markierten anti-Human-Immunglobulin oder dessen Fragmenten oder markiertem Protein A oder Protein G inkubiert werden und das von der gebundenen, markierten Substanz ausgesendete Signal bestimmt wird.

14. Verfahren nach Anspruch 12, **dadurch gekennzeichnet, dass** die Antikörper durch eine photometrische Messung der Extinktion nachwiesen werden, die durch die mit einem Enzym gekoppelten anti-Human-Antikörper oder deren Fragmente oder Protein A oder Protein G durch Spaltung eines geeigneten chromogenen oder fluorogenen Substrates verursacht wird.

15. Verfahren nach Anspruch 12, **dadurch gekennzeichnet, dass** die Antikörper durch eine Messung der Fluoreszenz nachgewiesen werden, die von der mit einer fluoreszierenden Gruppe markierte, gebundene Substanz verursacht wird.

16. Verfahren nach Anspruch 12, **dadurch gekennzeichnet, dass** man die Antikörper durch eine radiometrische Messung der mit einer radioaktiven Gruppe markierten, gebundene Substanz nachweist.

## Claims

1. Method for identifying an increased risk of the development and progression of arterial thromboses and atherosclerotic disorders and their sequelae such as, for example, angina pectoris, myocardial infarction and stroke, **characterized in that** in body fluids of a donor, especially in blood plasma, either
a) the heterozygous or homozygous occurrence of a mutant of the FSAP proenzyme with a G/A base exchange at nucleotide position 1601 is detected by analysis of the genomic DNA of the donor or in an mRNA derived therefrom,
**and/or**
b) the occurrence of an FSAP mutant which has lost the ability to activate single-chain plasminogen activators, or in which this ability is at least impaired, is detected at the protein level,
**and/or**
c) the occurrence of specific antibodies against an FSAP mutant which has lost the ability to activate single-chain plasminogen activators, or in which this ability is at least impaired, is detected,
**and/or**
d) a reduced potential of the FSAP to activate single-chain plasminogen activators is detected,
**and/or**
e) a reduced FSAP antigen concentration is detected.

2. Method according to Claim 1, **characterized in that** a heterozygous or homozygous occurrence of a mutant of FSAP is detected, where the mutant of FSAP has an amino acid exchange from glycine to glutamic acid at position 534 of the amino acid sequence of the FSAP proenzyme including the signal peptide.

3. Method according to Claim 1 and 2, **characterized in that** the heterozygous or homozygous occurrence of the mutant is detected by analysis of the sequence of the genomic DNA of a donor or of the mRNA derived therefrom in body fluids, with an amplification taking place with the aid of the polymerase chain reaction (PCR).

4. Method according to any of Claims 1 to 3, **characterized in that** a heterozygous or homozygous occurrence of a mutant of FSAP which has lost the ability to activate prourokinase, or in which this ability is at least impaired, is detected.

5. Method according to Claim 1 to 4, **characterized in that** the mutants of FSAP are detected at the protein level by using monoclonal or polyclonal antibodies.

6. Method according to Claim 5, **characterized in that** the mutants of FSAP are detected at the protein level by using monoclonal or polyclonal antibodies which are specific for the mutant.

7. Method according to Claim 5 and 6, **characterized in that** the mutants are detected by histological investigations on tissues or in solutions extracted from tissues.

8. Method according to Claim 1 to 7, **characterized in that** the detection of a reduced FSAP antigen concentration is carried out by means of an ELISA method, where FSAP from body fluids is bound as protein directly or indirectly via FSAP-binding substances immobilized on a solid phase, such as monoclonal or polyclonal antibodies, and their F(ab')₂ or Fab' fragments, as well as dextran sulphate or heparin, to a solid phase, and the determination of FSAP takes place either by determining the activity of the bound FSAP or by detecting the binding of further FSAP-specific binding partners.

9. Method according to Claim 1 to 8, **characterized in that** the detection of the FSAP mutant takes place by means of antibodies bound to a solid phase which is mixed with a sample to be investigated for the presence of the risk factor, in the presence of a labelled FSAP mutant, and the signal elicited by the label is measured.

10. Method according to Claim 1 to 9, **characterized in that** a reduced potential of FSAP to activate single-chain plasminogen activators is detected by measuring the activity of FSAP by incubating the protease-containing sample on a solid support onto which an antibody directed against the protease has previously been coupled and, after washing out the free support, incubating the protease immobilized thereon with reagents which allow determination of the activity thereof.

11. Method according to Claims 1 to 10, **characterized in that** antibodies are used on Western Blots for detecting the occurrence of the FSAP mutants for immunohistology or fluorescence-activated cell sorting (FACS).

12. Method according to Claims 1 to 11, **characterized in that** antibodies against a mutant of FSAP are detected by allowing a sample which might contain the antibodies against the mutant of FSAP to act on an FSAP mutant immobilized on a solid support, then washing and detecting the antibodies bound to the protease(s).

13. Method according to Claim 12, **characterized in that** the bound human antibodies are incubated with a labelled anti-human immunoglobulin or fragments thereof or labelled protein A or protein G, and the signal emitted by the bound, labelled substance is determined.

14. Method according to Claim 12, **characterized in that** the antibodies are detected by photometric measurement of the extinction caused by cleavage of a suitable chromogenic or fluorogenic substrate by the enzyme-coupled anti-human antibodies or fragments thereof or protein A or protein G.

15. Method according to Claim 12, **characterized in that** the antibodies are detected by a measurement of the fluorescence caused by the bound substance labelled with a fluorescent group.

16. Method according to Claim 12, **characterized in that** the antibodies are detected by a radiometric measurement of the bound substance labelled with a radioactive group.

## Revendications

1. Procédé pour l'identification d'un risque aggravé d'apparition et de progression des thromboses artérielles et des affections athérosclérotiques et de leurs complications telles que par exemple l'angine de poitrine, l'infarctus du myocarde et l'accident vasculaire cérébral, **caractérisé en ce qu'**on décèle dans des échantillons de liquides corporels d'un donneur, en particulier dans du plasma sanguin soit
a) la survenance hétérozygote ou homozygote d'un mutant de la proenzyme FSAP avec une substitution de bases G/A en position nucléotidique 1601, par l'analyse de l'ADN génomique du donneur ou dans un ARNm qui en est dérivé,
et/ou
b) la survenance au niveau protéique d'un mutant FSAP qui a perdu la capacité d'activation des activateurs du plasminogène simple chaîne, ou pour lequel cette capacité est au moins altérée,
et/ou
c) la présence d'anticorps spécifiques dirigés contre un mutant FSAP qui a perdu la capacité d'activation des activateurs du plasminogène simple chaîne, ou pour lequel cette capacité est au moins altérée,
et/ou
d) une diminution du potentiel de la FSAP pour l'activation des activateurs du plasminogène simple chaîne,
et/ou
e) une diminution de la concentration en antigènes de la FSAP.

2. Procédé selon la revendication 1, **caractérisé en ce qu'**on détecte une survenance hétérozygote ou homozygote d'un mutant de la FSAP, moyennant quoi le mutant de la FSAP présente une substitution aminoacide de la glycine vers l'acide glutamique en position 534 de la séquence aminoacide de la proenzyme FSAP, y compris du peptide signal.

3. Procédé selon les revendications 1 et 2, **caractérisé en ce que** la survenance hétérozygote ou homozygote du mutant est détectée dans des liquides corporels par l'analyse de la séquence de l'ADN génomique d'un donneur ou de l'ARNm qui en est dérivé, une amplification étant effectuée par réaction en chaîne par polymérase (PCR).

4. Procédé selon une des revendications 1 à 3, **caractérisé en ce qu'**on détecte une survenance hétérozygote ou homozygote d'un mutant de la FSAP qui a perdu la capacité d'activation de la prourokinase, ou pour lequel cette capacité est au moins altérée.

5. Procédé selon les revendications 1 à 4, **caractérisé en ce que** les mutants de la FSAP sont détectés au niveau protéique en utilisant des anticorps monoclonaux ou polyclonaux.

6. Procédé selon la revendication 5, **caractérisé en ce que** les mutants de la FSAP sont détectés au niveau protéique en utilisant des anticorps monoclonaux ou polyclonaux qui sont spécifiques du mutant.

7. Procédé selon les revendications 5 et 6, **caractérisé en ce que** les mutants sont détectés par des examens histologiques sur des tissus ou dans des solutions extraites de tissus.

8. Procédé selon les revendications 1 à 7, **caractérisé en ce que** la détection d'une diminution de la concentration en antigènes de la FSAP est effectuée au moyen d'un procédé ELISA, moyennant quoi la FSAP provenant de liquides corporels est liée à une phase solide sous forme de protéine directement ou indirectement par l'intermédiaire de substances liant la FSAP fixée à une phase solide, telles que des anticorps monoclonaux ou polyclonaux, ainsi que leurs fragments F(ab')2 ou Fab', mais aussi le sulfate de dextrane ou l'héparine, et la détermination de la FSAP liée est effectuée soit par la détermination de l'activité de la FSAP, soit par la détection de la liaison d'autres ligands spécifiques à la FSAP.

9. Procédé selon les revendications 1 à 8, **caractérisé en ce que** la détection du mutant FSAP est effectuée au moyen d'un anticorps lié à une phase solide qui est agglutiné à un échantillon à analyser quant à la présence d'un facteur de risque en présence d'un mutant FSAP marqué, et **en ce qu'**on mesure le signal engendré par le marquage.

10. Procédé selon les revendications 1 à 9, **caractérisé en ce que** pour la détection d'une diminution du potentiel de la FSAP pour l'activation des activateurs du plasminogène simple chaîne, on mesure l'activité de la FSAP en incubant l'échantillon contenant la protéase sur un support solide auquel a été précédemment couplé un anticorps dirigé contre la protéase, et après lavage du support libre, en incubant la protéase fixée sur le support avec des réactifs qui permettent la détermination de son activité.

11. Procédé selon les revendications 1 à 10, **caractérisé en ce que** pour la détection de la survenance des mutants FSAP, on utilise des anticorps sur des western blots d'immunohistologie, ou le tri cellulaire basé sur la fluorescence (FACS).

12. Procédé selon les revendications 1 à 11, **caractérisé en ce que** pour la détection d'anticorps dirigés contre un mutant de la FSAP, on laisse agir sur un mutant FSAP fixé à un support solide un échantillon susceptible de contenir les anticorps contre le mutant de la FSAP, après quoi on le lave et on détecte les anticorps liés à la/aux protéase(s).

13. Procédé selon la revendication 12, **caractérisé en ce que** les anticorps humains liés sont incubés avec une immunoglobuline anti-humaine marquée ou ses fragments, ou de la protéine A ou de la protéine G marquées, et on détermine le signal émis par la substance marquée liée.

14. Procédé selon la revendication 12, **caractérisé en ce que** les anticorps sont détectés par mesure photométrique de l'extinction qui est provoquée par les anticorps anti-humains couplés à une enzyme ou leurs fragments ou la protéine A ou la protéine G par le clivage d'un substrat chromogène ou fluorogène approprié.

15. Procédé selon la revendication 12, **caractérisé en ce que** les anticorps sont détectés par une mesure de la fluorescence qui est provoquée par la substance liée marquée avec un groupe fluorescent.

16. Procédé selon la revendication 12, **caractérisé en ce qu'**on détecte les anticorps par une mesure radiométrique de la substance liée marquée avec un groupe radioactif.
